# EUROPÄISCHE PATENTANMELDUNG

(11) **EP 1 086 668 A1**
(43) Veröffentlichungstag der Anmeldung: **28.03.2001**
(21) Anmeldenummer: 00810763.3
(22) Anmeldetag: 28.08.2000
(51) Int. Cl.: A61F 2/46

(54) **Setzinstrument für ein Tibiateil einer Kniegelenkprothese**

(30) Priorität: 21.09.1999 EP 99810841
(71) Anmelder: Sulzer Orthopedics Ltd., 6340 Baar (CH)
(72) Erfinder: Kohler, Michael, 8610 Uster (CH); Trachsler, Thomas, 8404 Winterthur (CH); Schwägerl, Wolfgang, Prof. Dr. med., 1160 Wien (AT); Böhler, Nikolaus, Prof. Dr. Med., 4020 Linz (AT)
(74) Vertreter: Sulzer Management AG

(57) **Zusammenfassung**

Ein Setzinstrument für ein Tibiateil (1) einer Kniegelenkprothese umfasst ein separates Ansatzstück (2) zur Befestigung an dem Tibiateil (1) und ein Handstück (3), welches mit dem separaten Ansatzstück (2) fest in Eingriff bringbar ist.

## Beschreibung

Die Erfindung betrifft ein Setzinstrument für ein Tibiateil einer Kniegelenkprothese.

Kniegelenkprothesen umfassen typischerweise ein Tibiateil, welches an der Tibia befestigt wird, sowie ein Femurteil, welches am Femur befestigt wird. Tibiateil und Femurteil sind regelmässig metallisch ausgebildet. Zwischen den kondylären Artikulationsflächen des Femurteils und der von der Tibia weg auf den Femur zu weisenden Oberfläche des Tibiateils ist typischerweise ein aus Kunststoff, z.B. aus Polyethylen, hergestellter Lagerkörper mit Lagerflächen angeordnet. Die kondylären Artikulationsflächen des Femurteils gleiten auf den Lagerflächen des Lagerkörpers.

Bei der Implantation einer Kniegelenkprothese werden nach der chirurgischen Eröffnung des Knies die Tibia und der Femur mit Hilfe von entsprechenden Lehren so präpariert, dass nach erfolgter Präparation die entsprechenden Prothesenteile, nämlich das Tibiateil einerseits und das Femurteil andererseits, an der Tibia bzw. am Femur befestigt werden können.

Die Implantation des Tibiateils erfolgt bisher typischerweise derart, dass der Orthopäde das Tibiateil mit der Hand auf der präparierten Tibia vorpositioniert bzw. leicht vorfixiert und dann den Tibiastamm oder einen mit dem Tibiastamm verbundenen Tibiaschaft weiter in die präparierte Tibia eintreibt. Dieses weitere Eintreiben erfolgt typischerweise mit Hilfe eines Instruments, welches auf die dem Femur zugewandte Fläche des Tibiateils aufgesetzt wird, dort jedoch nicht fixiert wird. Durch Schläge auf das proximale Ende dieses Instruments wird dann der Tibiastamm bzw. ein mit dem Tibiastamm verbundener Tibiaschaft in die Tibia eingetrieben, bis das Tibiateil in der gewünschten Weise an der Tibia befestigt ist.

Diese Art der Implantation des Tibiateils ist jedoch vom Handling her für den Orthopäden ziemlich unbequem. Zunächst muss er das Tibiateil per Hand auf der präparierten Tibia vorpositionieren und anschliessend mit einem nicht definitiv fixierbaren Instrument den Tibiastamm bzw. den mit dem Tibiastamm verbundenen Tibiaschaft weiter in die Tibia eintreiben. Beim Vorpositionieren besteht dabei die Gefahr, dass das Tibiateil dem Orthopäden oder anderem Operationspersonal aus der Hand gleitet und es in der Folge bei dieser Operation aus Sterilitätsgründen nicht mehr eingesetzt werden kann. Bei nicht-zentralem Aufsetzen des Instruments beim Eintreiben eines bereits vorpositionierten Tibiatiels kann es zu ungewolltem Kippen bzw. Verkanten des Tibiateils kommen, was dann beim weiteren Eintreiben entsprechend zu korrigieren ist.

Hier will die Erfindung Abhilfe schaffen. Insbesondere ist es eine Aufgabe der Erfindung, die oben genannten Nachteile zu vermeiden und dem Orthopäden ein Instrument an die Hand zu geben, welches ihm das Handling bei der Implantation erleichtert.

Diese Aufgabe wird durch ein Setzinstrument gelöst, wie es durch die Merkmale des unabhängigen Patentanspruchs charakterisiert ist. Besonders vorteilhafte Ausgestaltungen des Instruments ergeben sich aus den Merkmalen der abhängigen Patentansprüche.

Insbesondere wird die Aufgabe gelöst durch ein Setzinstrument für ein Tibiateil einer Kniegelenkprothese mit einem separaten Ansatzstück zur Befestigung an dem Tibiateil und mit einem Handstück, welches mit dem separaten Ansatzstück fest in Eingriff bringbar ist.

Vor der Implantation des Tibiateils wird also zunächst das Ansatzstück an dem Tibiateil befestigt und anschliessend wird das Handstück mit dem Ansatzstück fest in Eingriff gebracht. Diese Anordnung aus Tibiateil, Ansatzstück und Handstück bildet dann eine fest miteinander verbundene Einheit, die der Orthopäde sehr bequem handhaben kann. Die Anordnung ist für den Orthopäden sehr gut an dem Handstück zu greifen, sodass die Gefahr, dass das Tibiateil dem Orthopäden oder sonstigem Operationspersonal entgleitet, höchst gering ist. Weiterhin erlaubt das Setzinstrument aufgrund der guten Handhabbarkeit für den Orthopäden eine genaue Vorpositionierung des Tibiateils. Ist das Tibiateil vorpositioniert, so kann die Anordnung aus Tibiateil, Ansaztstück und Handstück fest miteinander verbunden bleiben und der Orthopäde kann - gegebenenfalls nach Kontrolle der Vorpositionierung - den Tibiastamm bzw. einen damit verbunden Tibiaschaft weiter in die Tibia eintreiben, ohne dabei das Instrument wechseln zu müssen. Er übt z.B. mit einem geeigneten Hammer Schläge auf das proximale Ende des Handstücks aus, wodurch der Tibiastamm bzw. ein damit verbundener Tibiaschaft weiter in die Tibia eingetrieben werden. Das Handling für den Orthopäden gestaltet sich dadurch einfach und bequem. Im übrigen sind sowohl das Ansatzstück als auch das Handstück einfach und zuverlässig sterilisierbar. Darüberhinaus erfolgt auch beim Eintreiben eine wohldefinierte Einleitung der Kräfte in das Tibiateil.

Bei einem Ausführungsbeispiel des Setzinstruments sind das Handstück und das Ansatzstück so ausgestaltet, dass sie im befestigten Zustand innerhalb des Bereichs zu liegen kommen, der von derjeinigen Fläche des Tibiateils begrenzt wird, die von der Tibia weg auf den Femur zuweist. Bei diesem Ausführungsbeispiel ragen also im assemblierten Zustand keine Instrumententeile über die "Aussenkonturen" des Tibiateils hinaus. Dies ist besonders für die Implantation von Vorteil, weil nämlich die chirurgische Eröffnung des Knies so gering wie möglich bleiben soll, andererseits aber Instrumententeile, die über die (maximalen äusseren) Abmessungen hinausstehen, beim Implantieren mit Weichteilen etc., die um die Tibia herum angeordnet sind, in räumlichen Konflikt kommen könnten und die Implantation behindern könnten.

Bei einem weiteren Ausführungsbeispiel weist das Ansatzstück ein Auflageteil auf, welches bei befestigtem Ansatzstück auf derjenigen Fläche des Tibiateils aufliegt, die von der Tibia weg auf den Femur zu weist, sowie ein Angriffsteil, welches proximal zum Auflageteil angeordnet ist und mit dem Handstück in Eingriff bringbar ist. Man könnte auch sagen, das Auflageteil liegt auf der Lagerfläche des Tibiateils auf und weist darüberhinaus ein proximal zu diesem Auflageteil angeordnetes Angriffsteil auf, die Auflage auf dem Tibiateil und der Eingriff mit dem Handstück sind somit voneinander getrennt.

Bei einer Weiterbildung ragt das Angriffsteil des Ansatzstücks von seinen seitlichen Abmessungen her betrachtet über das Auflageteil hinaus, sodass ein Vorsprung gebildet wird. Das Handstück ist an seinem distalen Ende mit einer Aussparung versehen, die beim Eingreifen von Handstück und Ansatzstück das Auflageteil des Ansatzstücks aufnimmt und dabei unter den Vorsprung greift. Diese Weiterbildung ermöglicht nach dem Befestigen des Ansatzstücks am Tibiateil ein einfaches Aufschieben des Handstücks, welches dann unter den Vorsprung greift. Durch anschliessendes Verspannen des Handstücks mit dem Ansatzstück kann dann auf einfache Weise die oben bereits beschriebene, fest miteinander verbundene Einheit aus Tibiateil, Ansatzstück und Handstück erstellt werden, die für den Orthopäden gut handhabbar ist. Diese für den Orthopäden gut handhabbare Einheit kann beispielsweise auch von anderem Operationspersonal zusammengestellt werden.

Bei einer Weiterbildung ist das Handstück mit einem Anschlag versehen, welcher beim Eingreifen von Handstück und Ansatzstück das Aufschieben des Handstücks in Richtung der Aussparung begrenzt. Dadurch wird verhindert, dass das Handstück zu weit unter den Vorsprung geschoben werden kann. Als Anschlag kann beispielweise ein entsprechend angeordneter Verbindungssteg vorgesehen sein.

Bei einem weiteren Ausführungsbeispiel des Setzinstruments sind zum Befestigen des Ansatzstücks am Tibiateil Mittel zum Verschrauben des Ansatzstücks mit dem Tibiateil vorgesehen. Dazu müssen natürlich am Tibiateil entsprechende Mittel (z.B. ein Gewinde) vorgesehen sein. Diese Art der Befestigung ist wenig aufwendig und auch vom Handling her einfach durchführbar.

Bei einem weiteren Ausführungsbeispiel weist das Handstück an seinem distalen Ende ein Eingriffsteil auf und proximal zum Eingriffsteil eine in Richtung auf das Eingriffsteil zu bzw. von diesem weg verstellbare Druckplatte. Die Druckplatte ist mit Druckstücken versehen, die fest mit der Druckplatte verbunden sind und die in entsprechenden Durchgangsbohrungen im Eingriffsteil des Handstücks angeordnet sind. Beim Verstellen der Druckplatte in Richtung auf das Eingriffsteil zu treten die Druckstücke am distalen Ende aus den Durchgangsbohrungen heraus bzw. beim Verstellen der Druckplatte gleiten sie in Richtung von dem Eingriffsteil weg in die Durchgangsbohrungen hinein. Zum festen Eingreifen des Handstücks mit dem am Tibiateil befestigten Ansatzstück wird also die Druckplatte auf das Eingriffsteil zu bewegt, was dadurch erfolgt, dass die Druckstücke am distalen Ende aus den Bohrungen austreten und sich auf der Lagerfläche des Tibiateils abstützen. Dadurch wird das Eingriffsteil von der Lagerfläche des Tibiateils abgehoben und greift fest unter den Vorsprung am Ansatzstück, wodurch eine feste Anordnung bestehend aus Tibiateil, Ansatzstück und Handstück gebildet wird. Nach dem Eintreiben des Tibiateils wird das Druckstück wieder von dem Eingriffsteil weg bewegt, wobei die Druckstücke in die Bohrungen im Eingriffsteil zurück gleiten und das Eingriffsteil wieder auf der Tibialagerfläche zu liegen kommt, und nur locker unter den Vorsprung greift, sodass das Handstück wieder entfernt werden kann. Anschliessend wird das Ansatzstück vom Tibiateil entfernt und die Implantation des Tibiteils ist abgeschlossen. Dieses Ausführungsbeispiel ist sowohl vom konstruktiven Aufwand als auch vom Handling her einfach.

Um das Tibiateil einzutreiben, kann am proximalen Ende des Handstücks eine Schlagfläche vorgesehen sein. Auf diese Schlagfläche können Schlagimpulse mit einem geeigneten Instrument, beispielsweise einem Hammer, ausgeübt werden, um den Tibiastamm bzw. einen mit dem Tibiastamm verbundenen Schaft in die Tibia einzutreiben.

Im folgenden wird die Erfindung anhand der Zeichnung näher erläutert. Dabei zeigen in schematischer Darstellung:
- Fig. 1: eine Explosionsdarstellung der einzelnen Komponenten: Tibiateil mit Tibiastamm, Schaft zur Aufnahme im Tibiastamm, Dehnschraube zur Verbindung von Schaft und Tibiastamm, Ansatzstück und Handstück,
- Fig. 2: die Explosionsdarstellung aus Fig. 1, jedoch aus einer anderen Perspektive,
- Fig. 3: ein Tibiateil mit bereits am Tibiastamm befestigtem Tibiaschaft und mit einem am Tibiateil befestigten Ansatzstück einerseits, sowie das Handstück andererseits,
- Fig. 4: eine vollständig assemblierte Anordnung umfassend ein Tibiateil mit bereits am Tibiastamm befestigtem Tibiaschaft, mit einem am Tibiateil befestigten Ansatzstück, sowie mit einem mit dem Ansatzstück in festem Eingriff befindlichen Handstück
und
- Fig. 5: die vollständig assemblierte Anordnung aus Fig. 4 in einer anderen Ansicht.

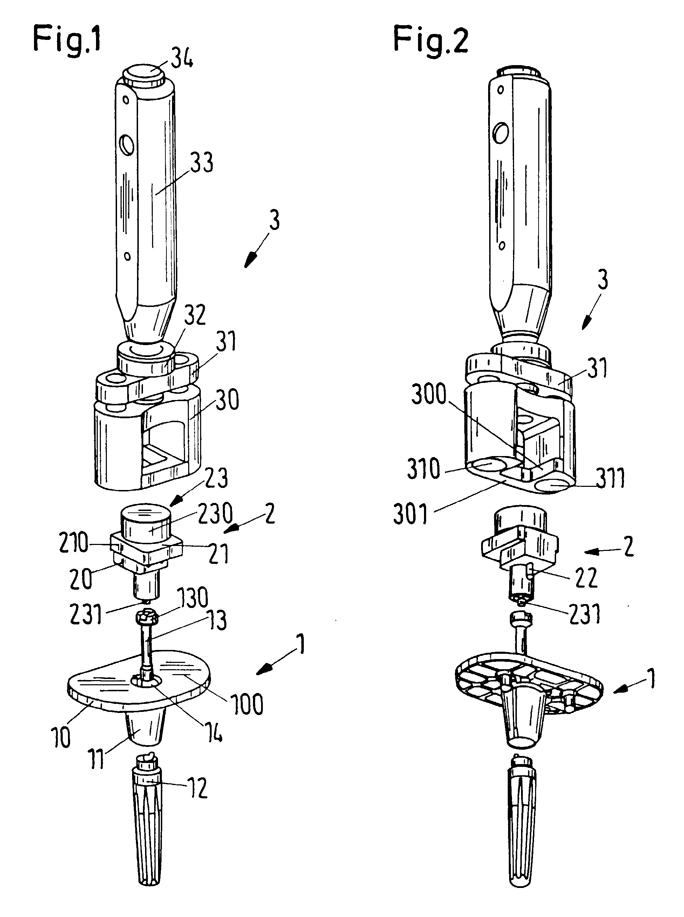

In der Explosionsdarstellung in Fig. 1 erkennt man ein Tibiateil 1, welches hier ein Tibiaplateau 10 mit einem Tibiastamm 11 umfasst, sowie ein Tibiaschaft 12, welcher mit Hilfe einer Dehnschraube 13, die durch eine Bohrung 14 hindurch den Tibiaschaft 12 mit dem Tibiastamm 11 und damit mit dem Tibiaplateau 10 verbindet. Im Kopf 130 der Dehnschraube 13 ist ein Gewinde vorgesehen, auf dessen Funktion weiter unten noch genauer eingegangen wird.

Ferner erkennt man in Fig. 1 ein Ansatzstück 2, welches an dem Tibiateil 1 befestigbar ist. Das Ansatzstück 2 weist ein Auflageteil 20 auf, welches im befestigten Zustand auf der Lagerfläche 100 des Tibiaplateaus 10 aufliegt. Ferner weist das Ansatzstück 2 ein Angriffsteil 21 auf, welches proximal zum Auflageteil 20 angeordnet ist und von seinen seitlichen Abmessungen her über das Auflageteil 20 hinausragt. Dadurch wird ein Vorsprung 210 gebildet, dessen Funktion später noch erläutert wird. Ferner erkennt man in Fig. 2, dass das Ansatzstück 2 eine Drehsicherung in Form eines nach unten vom Auflageteil 20 abstehenden Stifts 22 aufweist, welcher in eine entsprechende Ausnehmung in der Bohrung 14 im Tibiateil 1 eingreift. Schliesslich weist das Ansatzstück 2 noch eine Schraube 23 auf, welche einen Schraubenkopf 230 zum Bedienen der Schraube 23 aufweist, sowie einen Gewindeschaft 231, der am unteren Ende des Ansatzstücks 2 herausragt. Dieser Gewindeschaft 231 ist dazu vorgesehen, in das Gewinde im Kopf 130 der Dehnschraube 13 einzugreifen.

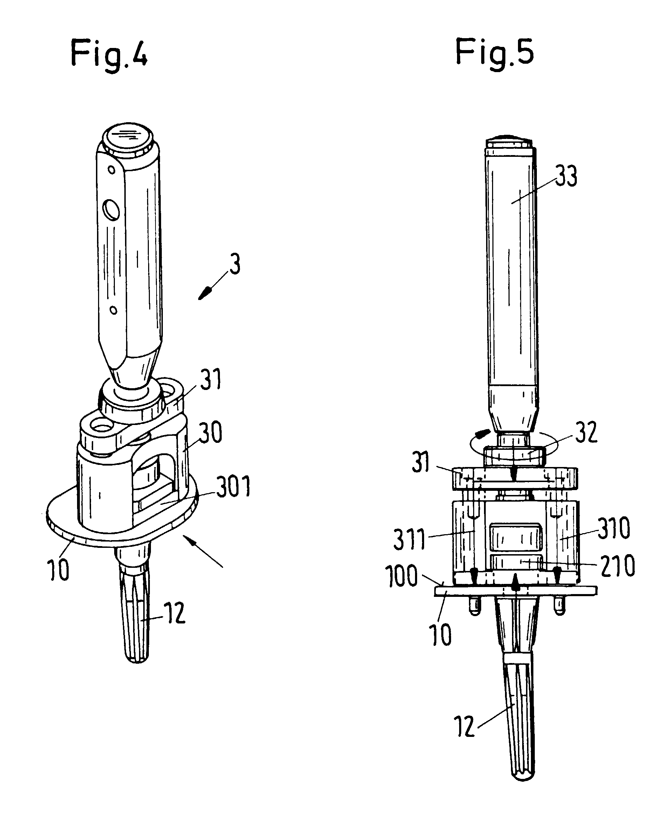

Schliesslich erkennt man in Fig. 1 noch das Handstück 3, welches an seinem distalen Ende ein Eingriffsteil 30 umfasst, sowie proximal dazu eine Druckplatte 31, und ferner eine Verstellschraube 32 sowie einen Handgriff 33, an dessen proximalem Ende eine Schlagfläche 34 vorgesehen ist. Zwischen dem Eingriffsteil 30 und der Druckplatte 31 sind Rückstellfedern (nicht dargestellt) angeordnet. Die Druckplatte 31 ist mit Hilfe der Verstellschraube 32 in Richtung auf das Eingriffsteil 31 zu bzw. in Richtung vom Eingriffsteil 31 weg verstellbar. Die Druckplatte ist mit zwei Druckstücken 310 und 311 versehen (Fig. 2), die fest mit der Druckplatte 31 verbunden sind (z.B. mit dieser verschraubt sind, siehe Fig. 5).

In Fig. 2 erkennt man noch, dass das Eingriffsteil 30 an seinem distalen Ende eine Aussparung 300 aufweist, welche einseitig durch einen quer zur Aussparung verlaufenden Steg 301 geschlossen ist. Der Steg 301 bildet dabei einen Anschlag, wie nachfolgend noch bei der Beschreibung der Funktionsweise des Instruments erläutert werden wird

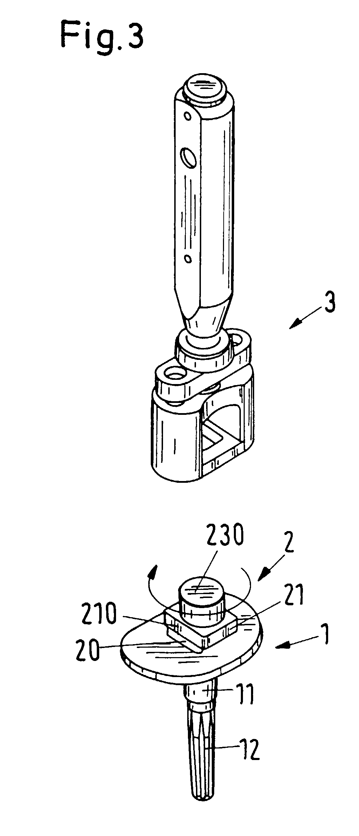

Die Funktionsweise ist wie folgt: Bei am Tibiaplateau 10 befestigtem Tibiaschaft 12 (ein Tibiaschaft 12 wird nicht bei jeder Implantation benötigt, insbesondere nach Möglichkeit nicht bei Erstimplantationen, kommt jedoch speziell bei Reoperationen oder schlechter Knochenbeschaffenheit regelmässig zum Einsatz) wird das Ansatzstück 2 an dem Tibiateil 1 befestigt, indem durch Verdrehen des Schraubenkopfs 230 (siehe Pfeil in Fig. 3) der Gewindeschaft 231 (Fig. 2) in das Gewinde im Kopf 130 (Fig. 1) der Dehnschraube 13 hineingeschraubt wird. Alternativ ist im Tibiastamm 11 ein Gewinde vorgesehen für den Fall, dass kein Tibiaschaft 12 benötigt wird, und der Gewindeschaft 231 der Schraube 23 ist dann entsprechend dicker ausgebildet, weil er dann direkt in das Gewinde im Tibiastamm 11 eingeschraubt wird, da ja keine Dehnschraube 13 benötigt wird. Der Stift 22 (Fig. 2) verhindert, dass das Ansatzstück 2 sich beim Verschrauben verdrehen kann.

Ist das Ansatzstück 2 mit dem Tibiateil 1 verschraubt (Fig. 3), so wird anschliessend das Handstück 3 mit seinem Eingriffsteil 30 so aufgeschoben (siehe Pfeil in Fig. 4), dass die Aufnahme 300 (siehe Fig. 2) das Auflageteil 20 des Ansatzstücks 2 aufnimmt und locker unter den überstehenden Vorsprung 210 greift, der durch das Angriffsteil 21 des Ansatzstücks 2 gebildet wird. Das Aufschieben des Handstücks 3 erfolgt dabei so lange, bis der Steg 301 (siehe Fig. 2) an dem Auflagestück 20 anstösst (siehe Fig. 4), der Steg 301 bildet also einen Anschlag für das Aufschieben des Handstücks 3 bzw. für dessen Eingriffsteil 30.

Ist das Handstück 3 bzw. sein Eingriffsteil 30 vollständig aufgeschoben (Fig.4), so greift es - wie bereits erwähnt - locker unter den Vorsprung 210 des Ansatzstücks 2, ist aber noch nicht fest mit diesem verbunden. Nun wird die Verstellschraube 32 betätigt, wodurch die Druckplatte 31 mit den daran befestigten Druckstücken 310 und 311 in Richtung auf das Tibiaplateau 10 zu bewegt wird (siehe Pfeile in Fig. 5). In der Folge treten die Druckstücke 310 und 311 am distalen Ende aus den Bohrungen im Eingriffsteil 30 aus (siehe Pfeile in Fig. 5) und stützen sich auf dem Tibiaplateau 10, genauer gesagt auf der Lagerfläche 100 des Tibiaplateaus 10, ab. Die Druckstücke 310 und 311 sind fest mit der Druckplatte 31 verbunden, sie sind beispielsweise - wie dies in Fig. 5 angedeutet ist - mit der Druckplatte 31 verschraubt. Andererseits ist das Eingriffsteil 30 unbeweglich mit dem Handgriff 33 bzw. mit dem Schaft verbunden, entlang welchem die Verstellschraube 32 verstellbar ist. Durch das Verstellen der Druckplatte 31 in Richtung auf das Eingriffsteil 30 zu und das dadurch erfolgende Austreten der Druckstücke 310 und 311 am distalen Ende des Eingriffsteils 30 wird also das Eingriffsteil 30 nach oben gezogen (siehe Pfeil in Fig. 5) und greift fest unter den Vorsprung 210 (sieh Fig. 2), der durch das Angriffsteil 21 des Ansatzstücks 2 gebildet wird. Dadurch kommt eine feste Verbindung von Handstück 3, Ansatzstück 2 und Tibiateil 1 zustande.

Diese Handlungen müssen nicht von dem Orthopäden selbst vorgenommen werden, sondern können zum Beispiel von anderem bei der Operation anwesenden Operationspersonal vorgenommen werden. Die in Fig. 5 dargestelle, fest miteinander verbundene Anordnung aus Tibiateil 1, Ansatzstück 2 und Handstück 3 kann dann dem Orthopäden zur Implantation übergeben werden. Der Orthopäde übernimmt diese und hat dann für die Implantation eine einfach zu handhabende und sicher miteinander verbundene Anordnung in der Hand.

Ist das Tibiateil 1 implantiert, so wird anschliessend die Verstellschraube 23 derart verstellt, dass sich die Druckplatte 31 wieder in Richtung von dem Eingriffsteil 30 weg bewegt. Sodann drücken die Rückstellfedern (nicht dargestellt) das Eingriffsteil 30 von dem Druckstück 31 weg, wodurch die Druckstücke 310 und 311 wieder in die Bohrungen im Eingriffsteil 30 hinein gleiten. Dadurch wird der feste Eingriff von Handstück 3 und Ansatzstück 2 wieder gelöst und das Handstück 3 kann abgenommen, gereinigt und sterilisiert werden. Anschliessend kann das Ansatzstück 2 vom Tibiateil 1 abgeschraubt werden (durch Drehen des Schraubenkopfs 230) und die Implantation des Tibiateils 1 ist abgeschlossen.

## Patentansprüche

1. Setzinstrument für ein Tibiateil (1) einer Kniegelenkprothese, mit einem separaten Ansatzstück (2) zur Befestigung an dem Tibiateil (1) und mit einem Handstück (3), welches mit dem separaten Ansatzstück (2) fest in Eingriff bringbar ist.

2. Setzinstrument nach Anspruch 1, bei welchem das Handstück (3) und das Ansatzstück (2) so ausgestaltet sind, dass sie im befestigten Zustand innerhalb des Bereichs zu liegen kommen, der von derjeinigen Fläche (12) des Tibiateils (1) begrenzt wird, die von der Tibia weg auf den Femur zuweist.

3. Setzinstrument nach einem der vorangehenden Ansprüche, bei welchem das Ansatzstück (2) ein Auflageteil (20) aufweist, welches bei befestigtem Ansatzstück (2) auf derjenigen Fläche (100) des Tibiateils (1) aufliegt, die von der Tibia weg auf den Femur zu weist, sowie ein Angriffsteil (21), welches proximal zum Auflageteil (20) angeordnet ist und mit dem Handstück (3) in Eingriff bringbar ist.

4. Setzinstrument nach Anspruch 3, bei welchem das Angriffsteil (21) des Ansatzstücks (2) von seinen seitlichen Abmessungen her betrachtet über das Auflageteil (20) hinausragt, sodass ein Vorsprung (210) gebildet wird, und bei welchem das Handstück (3) an seinem distalen Ende mit einer Aussparung (300) versehen ist, die beim Eingreifen von Handstück (3) und Ansatzstück (2) das Auflageteil (20) des Ansatzstücks (2) aufnimmt und dabei unter den Vorsprung (210) greift.

5. Setzinstrument nach Anspruch 4, bei welchem das Handstück (3) mit einem Anschlag (301) versehen ist, welcher beim Eingreifen von Handstück und Ansatzstück das Aufschieben des Handstücks (3) in Richtung der Aussparung (300) begrenzt.

6. Setzinstrument nach einem der vorangehenden Ansprüche, bei welchem zum Befestigen des Ansatzstücks (2) am Tibiateil (1) Mittel zum Verschrauben (23) des Ansatzstücks (2) mit dem Tibiateil (1) vorgesehen sind.

7. Setzinstrument nach einem vorangehenden Ansprüche, bei welchem das Handstück (3) an seinem distalen Ende ein Eingriffsteil (30) aufweist und proximal zum Eingriffsteil (30) eine in Richtung auf das Eingriffsteil (30) zu bzw. von diesem weg verstellbare Druckplatte (31) aufweist, wobei die Druckplatte (31) mit Druckstücken (310,311) versehen ist, die fest mit der Druckplatte (31) verbunden sind und die in entsprechenden Durchgangsbohrungen im Eingriffsteil (30) des Handstücks (3) angeordnet sind, sodass die Druckstücke (310,311) beim Verstellen der Druckplatte (31) in Richtung auf das Eingriffsteil (30) zu am distalen Ende aus den Durchgangsbohrungen heraustreten bzw. beim Verstellen der Druckplatte (31) in Richtung von dem Eingriffsteil (30) weg in die Durchgangsbohrungen hineingleiten.

8. Setzinstrument nach einem der vorangehenden Ansprüche, bei welchem am proximalen Ende des Handstücks (3) eine Schlagfläche (34) vorgesehen ist.
